Europäisches Patentamt

European Patent Office

Office européen des brevets

Publication number: **0 285 214**
A1

# EUROPEAN PATENT APPLICATION

(21) Application number: 88200546.5

(22) Date of filing: 23.03.88

(51) Int. Cl.⁴: **A61B 6/14**

(30) Priority: 30.03.87 NL 8700733

(43) Date of publication of application:
05.10.88 Bulletin 88/40

(84) Designated Contracting States:
DE FR GB IT NL

(71) Applicant: N.V. Philips' Gloeilampenfabrieken
Groenewoudseweg 1
NL-5621 BA Eindhoven(NL)

(72) Inventor: Schuppert, Cornelis Leendert
c/o INT. OCTROOIBUREAU B.V. Prof.
Holstlaan 6
NL-5656 AA Eindhoven(NL)

(74) Representative: Scheele, Edial François et al
INTERNATIONAAL OCTROOIBUREAU B.V.
Prof. Holstlaan 6
NL-5656 AA Eindhoven(NL)

(54) Dental X-ray apparatus.

(57) A dental X-ray fluoroscopy apparatus comprises a fibre optical system (28) which is curved through 180° and which includes an entrance window (36) for intra-oral use. Because use is made of such a fibre optical system, the X-ray source (2) and the detector (14) can be accommodated together in one housing (1). Via three rotation couplings (42, 45 and 48), the housing is connected to an apparatus carrier (49). The use of a semiconductor CCD matrix (76) in the detection device (12) and a suitably chosen centre of gravity (50) for the apparatus with respect to at least the central rotation coupling (45), make the apparatus light and easy to position.

FIG.1

## "Dental X-ray apparatus."

The invention relates to a dental X-ray fluoroscopy apparatus, comprising a detector device and an X-ray source which is directed thereto.

An apparatus of this kind is known from EP 149502. The X-ray tube and the detector of an apparatus described therein are mounted at an angle of approximately 90° with respect to one another and are interconnected by way of a comparatively long bracket. As a result, the ease of operation and adjustability of the apparatus are less than desirable. Moreover, the construction is not very compact and the risk of disorientation of the source with respect to the detector device exists.

A more compact construction is obtained in an apparatus described in EP 129938. In this apparatus, however, the arrangement of the X-ray source is intra-oral and the detector is arranged so as to be extra-oral. It is a drawback of this solution that, due to the positioning of the X-ray source or rather of a focal point thereof with respect to an object to be examined, imaging will not be optimum in all circumstances. The orientation of the X-ray tube and an entrance face of the detector device with respect to one another cannot always be exactly adjusted in practice, so that imaging errors still occur.

It is the object of the invention to mitigate these drawbacks; to achieve this, a dental X-ray apparatus of the kind set forth in accordance with the invention is characterized in that an entrance face of the detector device is oriented transversely of an X-ray beam to be emitted by the X-ray source and is optically coupled to an entrance face of an image intensifier by way of a substantially 180° curved fibre optical system.

Because the X-ray beam in an apparatus in accordance with the invention is always directed transversely of an entrance face of the detector device, optimum imaging is always ensured whilst, as result of the 180° deflection via the fibre optical system, an extremely compact construction can still be obtained.

The X-ray source and the detector in a preferred embodiment are accommodated together in a housing, so that an extremely compact and readily adjustable apparatus is obtained. Longitudinal axes of an X-ray tube and an image intensifier tube then preferably extend in parallel. An arm which emerges from the rear of the housing and which preferably enables rotation about said longitudinal axes as well as a translation of the apparatus, can provide connection to, for example a mobile carrier, a patient carrier or a stationary wall.

Using such a link, a centre of gravity of the apparatus preferably occupies a fixed position with respect to a rotary axis of the arm.

In a further preferred embodiment, the fibre optical system is constructed as a rigid bar which is rigidly connected to the housing, an image intensifier of the detector device being resiliently clamped against an end of the fibre bar. The fibre optical system preferably consists of an optical fibre bar which comprises continuous fibres and which has been curved through 180°, but may also be composed of a plurality of parts which interrupt the fibres in a longitudinal direction. If geometrical adaptation of the fibre bar to an entrance face of an image intensifier is desirable, the fibre bar may comprise appropriately adapted conical parts.

In a further preferred embodiment, an X-ray diaphragm in the form of a conical adapter is included in order to mark a radiation field which is adapted to the object to be examined.

Some preferred embodiments in accordance with the invention will be described in detail hereinafter with reference to the drawing; therein:

Figure 1 is a diagrammatic view of a dental apparatus in accordance with the invention, and

Figure 2 is an exploded view thereof.

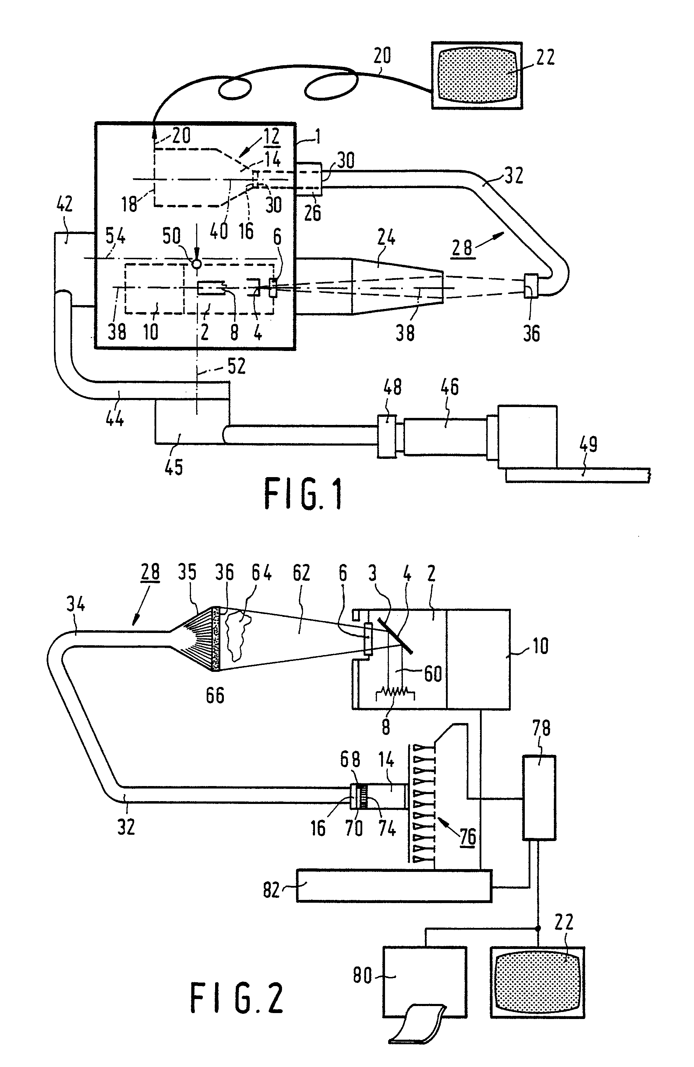

A dental X-ray fluoroscopy apparatus as shown in Figure 1 comprises a housing 1 which accommodates an X-ray source 2 having a radiation focus 4, an exit window 6, a cathode 8 and a power supply unit 10, and a detector device 12 with an image intensifier device 14 having an image entrance face 16 and an image exit face 18 wherefrom image signals can be applied to a monitor 22 via a cable 20.

Proceeding in the direction away from the exit window 6 of the X-ray tube, the housing comprises a conical radiation limiter 24 for marking a radiation field. Opposite the entrance face 16 of the image intensifier 14 there is arranged a holder 26 for a fibre optical system 28. An exit face 30 of the fibre optical system is optically coupled to the entrance face 16 of the image intensifier. The image intensifier is notably clamped resiliently against the exit face 30 and the fibre optical system is rigidly connected to the housing via the holder 26.

Via, for example two curves 32 and 34, the fibre optical system is curved through 180°, so that an entrance face 36 thereof is situated in the prolongation of a longitudinal axis 38 of the X-ray tube. Thus, an optical coupling is realized between the X-ray tube axis 38 of the X-ray tube and hence a main ray of the imaging X-ray beam and the optical axis 40 of the image intensifier. Via a rotation coupling 42, a carrier arm 44 is secured to the housing, which arm in this case comprises a second rotation coupling 45 and a telescopic arm 46.

Via a third rotation coupling 48 in the telescopic arm, the housing can be connected to, for example a mobile carrier, a patient carrier or a stationary wall (49). The arm and the apparatus are preferably constructed so that a centre of gravity 50 of the apparatus is situated near or on an axis of rotation 52 of the second rotation coupling 45 and preferably also on or near an axis of rotation 54 of the rotation coupling 42. The apparatus can be simply positioned by means of the three-fold rotatable and translatable arm.

Figure 2 separately shows the various parts of the apparatus. For the sake of clarity, the scale is substantially disturbed. An electron beam 60 emerging from the cathode 8 of the X-ray tube 2 is incident on an anode 3, a target anode 4 and generates an X-ray beam 62 which strikes, via the exit window 6 of the X-ray tube, an object to be examined, for example a molar 64 and which projects an image thereof onto the entrance screen 36 of the fibre optical system 28. The entrance face of the fibre optical system is provided with a luminescent layer 66, for example a vapour-deposited layer of CsI (Th) having a thickness of, for example 50-300 μm. A light-optical image produced therein by the X-rays is projected onto the entrance face 16 of the image intensifier 14 via the fibre optical system 28. A photo-cathode 68 provided on an entrance window 70 of the image intensifier tube converts the light-optical image into an image-carrying photo-electron beam which is intensified by a channel plate intensifier 74 and converted into a photon beam which strikes a CCD matrix 76. Signals of the CCD matrix which may comprise, for example between 200 × 200 and 600 × 600 elements, are applied to a, for example 8-bit, memory 78 and subsequently to the monitor 22 and/or a hard copy unit 80.

A central power supply and control device 82 controls and powers the X-ray tube, the channel plate intensifier, the CCD matrix and the memory.

Because of the high intensification, for example a factor $10^4$ with a comparatively simple power supply source, an image intensifier comprising a channel plate intensifier is more attractive than an acceleration-type electron-optical tube. In addition to the higher intensification, the channel plate intensifier offers the advantage of low weight and small volume.

The fibre optical system 28 in the present embodiment comprises a conical portion 35 at the entrance side. If desirable, the end may also be constructed so as to be conical, but the entrance face of a channel plate CCD intensifier is usually so small that this will not be necessary. The fibre optical system preferably consists of a single bar of continuous glass fibres which is bent through an angle of 180°, said fibres being stacked with a pitch of from 5 to 10 μm.

## Claims

1. A dental X-ray fluoroscopy apparatus, comprising a detector device and an X-ray source which is directed thereto, characterized in that an entrance face 36 of the detector device is oriented transversely of an X-ray beam 62 to be emitted by the X-ray source 2 and is optically coupled to an entrance face 16 of an image intensifier 14 of the detector device, by way of a substantially 180° curved fibre optical system 28.

2. A dental X-ray apparatus as claimed in Claim 1, characterized in that the X-ray source and the image intensifier are accommodated in a common housing (1).

3. A dental X-ray apparatus as claimed in Claim 2, characterized in that the housing is connected to a carrier (49) via a carrier arm (44) comprising three rotation couplings (42, 45, 48).

4. A dental X-ray apparatus as claimed in Claim 3, characterized in that a centre of gravity (50) of the apparatus occupies a permanently selected position with respect to a rotatable coupling (45) of the carrier arm (44), which rotatable coupling is not rigidly connected to the housing or the carrier.

5. A dental X-ray apparatus as claimed in Claim 3 or 4, characterized in that the centre of gravity of the apparatus is situated on or near a rotary axis of a housing/arm coupling (42).

6. A dental X-ray apparatus as claimed in any one of the preceding Claims, characterized in that the fibre optical system is formed by a rigid fibre optical bar which is rigidly connected to a housing for the image intensifier device.

7. A dental X-ray apparatus as claimed in Claim 5, characterized in that an image window of the detection device is resiliently coupled to an exit face of the fibre optical system.

8. A dental X-ray apparatus as claimed in any one of the preceding Claims, characterized in that the fibre optical system is composed of a plurality of parts having a conical shape or not.

9. A dental X-ray apparatus as claimed in any one of the preceding Claims, characterized in that the detector device comprises a channel plate intensifier (74) and a CCD television pick-up element (76).

10. A dental X-ray apparatus as claimed in any one of the preceding Claims, characterized in that the detector device comprises an integrated fibre optical system (28), photo-cathode (60), channel plate intensifier (74) and CCD detection matrix (76).

FIG.1

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | US-A-4 259 583 (R.D. ALBERT)<br>* Abstract; column 4, line 59 - column 5, line 54; column 5, line 64 - column 6, line 8; column 7, lines 31-38; column 7, line 59 - column 8, line 7; figures 1-4 *<br>--- | 1-3,8 | A 61 B    6/14 |
| X | US-A-3 790 785 (F. PAOLINI et al.)<br>* Abstract; column 1, line 65 - column 2, line 10; column 4, lines 3-23; figures 1-4 *<br>--- | 1,2,6 | |
| D,A | EP-A-0 149 502 (PHILIPS)<br>* Abstract; page 3, lines 7-11,17-20; page 8, lines 20-25,29-31; page 9, lines 20-33; page 10, lines 7-29; page 11, lines 21-33; figures 1-6 *<br>--- | 1,3,7,8 | |
| A | EP-A-0 129 451 (F. MOUYEN)<br>* Abstract; page 3, lines 12-36; page 4, lines 18-28; page 5, lines 18-35; page 6, lines 16-24; page 9, lines 12-16; figure 1 *<br>--- | 1,3,8-10 | TECHNICAL FIELDS SEARCHED (Int. Cl.4) |
| A | DE-U-7 525 125 (SIEMENS)<br>* Page 3, lines 15-27; page 4, lines 7-29; figures 1-4 *<br>--- | 3-5 | A 61 B |
| D,A | EP-A-0 129 938 (PHILIPS)<br>* Abstract; page 3, lines 8-33; page 4, lines 16-19; figure 1 *<br>----- | 1,3,8 | |

The present search report has been drawn up for all claims

| Place of search | Date of completion of the search | Examiner |
|---|---|---|
| THE HAGUE | 08-07-1988 | RIEB K.D. |